# EUROPEAN PATENT SPECIFICATION

(11) **EP 0 456 736 B1**
(45) Date of publication and mention of the grant of the patent: **19.07.1995**
(21) Application number: 90903064.5
(22) Date of filing: 19.01.1990
(51) Int. Cl.: C08F 265/02, C08K 7/02

(54) **AGGREGATES OR CLUSTERS OF WATER-SWELLABLE POLYMERS HAVING INCREASED HYDRATION RATE OVER UNASSOCIATED WATER-SWELLABLE POLYMERS**
AGGREGATE ODER CLUSTER VON WASSERQUELLFÄHIGEN POLYMEREN MIT ERHÖHTER HYDRATATIONSGESCHWINDIGKEIT GEGENÜBER NICHTASSOZIIERTEN WASSERQUELLFÄHIGEN POLYMEREN
AGREGATS OU AGGLOMERATS DE POLYMERES GONFLANT A L'EAU AYANT UN TAUX D'HYDRATATION SUPERIEUR A CELUI DE POLYMERES GONFLANT A L'EAU NON ASSOCIES

(30) Priority: 24.01.1989 US 304616
(43) Date of publication of application: 21.11.1991
(73) Proprietor: THE DOW CHEMICAL COMPANY, Midland, Michigan 48640 (US)
(72) Inventor: TAI, Eva, F., Midland, MI 48640 (US)
(74) Representative: Sternagel, Hans-Günther, Dr.
(86) International application number: US9000413
(87) International publication number: WO9008789

(56) References cited:
- EP-A- 0 233 014
- US-A- 4 131 576
- US-A- 4 191 672
- US-A- 4 446 261
- US-A- 4 647 617
- US-A- 4 708 997
- US-A- 4 735 987

## Description

Water-swellable polymers are well known in the art as useful in diapers and various other devices where aqueous absorption is desired. Often during the production of such polymers, a variety of particle sizes are produced. Those particles of very fine particle size, smaller than 88 micrometers (170 mesh), do not absorb moisture as desirably as larger polymer particles. These fines are often separated and discarded from the desirable larger particles. The problem with the finer particles is thought to be gel blocking, whereby an aqueous solution does not have access to the polymer because of dense packing of the particles and surface gelling of the polymer mass.

Therefore, it would be desirable to create a method whereby such fine particles could be recycled or reforged into useful water-absorbing polymer particles rather than being discarded.

EP-A-233 014 discloses a process for preparing agglomerates of water-swellable polymeric material by forming a dispersion in non-aqueous liquid of seed particles of water-swellable cross linked polymer, mixing into the dispersion an aqueous solution of water soluble ethylenically unsatured second monomer and polymerizing the second monomer to form the agglomerates.

The present invention is a method for preparing aggregates or clusters of water-swellable polymers, having improved hydration rates and absorbing behavior.

The present invention is a process for preparing water-swellable polymer clusters comprised of water-swellable polymer particles being associated to each other in a random packing configuration and spatially distributed, which process comprises:
(a) suspending water-swellable polymer particles in an inert hydrophobic liquid to form a first suspension;
(b) suspending an amorphous highly oil-dispersible substantially water-insoluble particulate material in an inert hydrophobic liquid and adding an aqueous solution comprising an ethylenically unsaturated carboxylic acid monomer that is polymerizable with the water-swellable polymer to form a second suspension; and
(c) slowly adding said second suspension to said first suspension, while said first suspension is agitated and is exposed to polymerization conditions such that said particles are bonded to each other, whereby clusters of said water-swellable particles are formed.

Optionally, additional steps may include
(d) drying the aggregates and (e) crushing the aggregates.

The present invention further relates to an absorbant article, comprising:
(a) 50 to 98 percent by weight of said article of a hydrophilic fiber material; and
(b) 50 percent to 2 percent by weight of said article of aggregates of water-swellable polymers, prepared by (i) suspending water-swellable polymer particles in an inert hydrophobic liquid to form a first suspension; (ii) suspending an amorphous highly oil-dispersible substantially water-insoluble particulate material in an inert hydrophobic liquid and adding an aqueous solution comprising an ethylenically unsaturated carboxylic acid monomer that is polymerizable with the water-swellable polymer to form a second suspension; (iii) slowly adding said second suspension to said first suspension, while said first suspension is agitated and is exposed to polymerization conditions such that said particles are bonded to each other; and (iv) drying said aggregate.

The present invention also includes aggregates or clusters of water-swellable polymers, said aggregates having improved races of hydration and absorbing behavior, characterized by: water-swellable polymer particles associated by bonding to other water-swellable polymer particles in a random packing configuration spacially distributed to allow aqueous absorption. The term "aggregates" in this application means discrete clusters of water-swellable polymer particles. Permeated with many channels wherein a contacting aqueous fluid has access to and is absorbed by said polymer.

The aggregates or clusters of the invention are typically comprised of water-swellable polymer particles having a broad range of unassociated particle sizes of 841 micrometers (20 mesh) to 37 micrometers (400 mesh). The water-swellable polymer particles are associated or clustered by bonding to other water-swellable polymer particles in a random packing configuration spacially distributed to allow aqueous absorption, said aggregates or clusters having a size of 300 micrometers to 3000 micrometers.

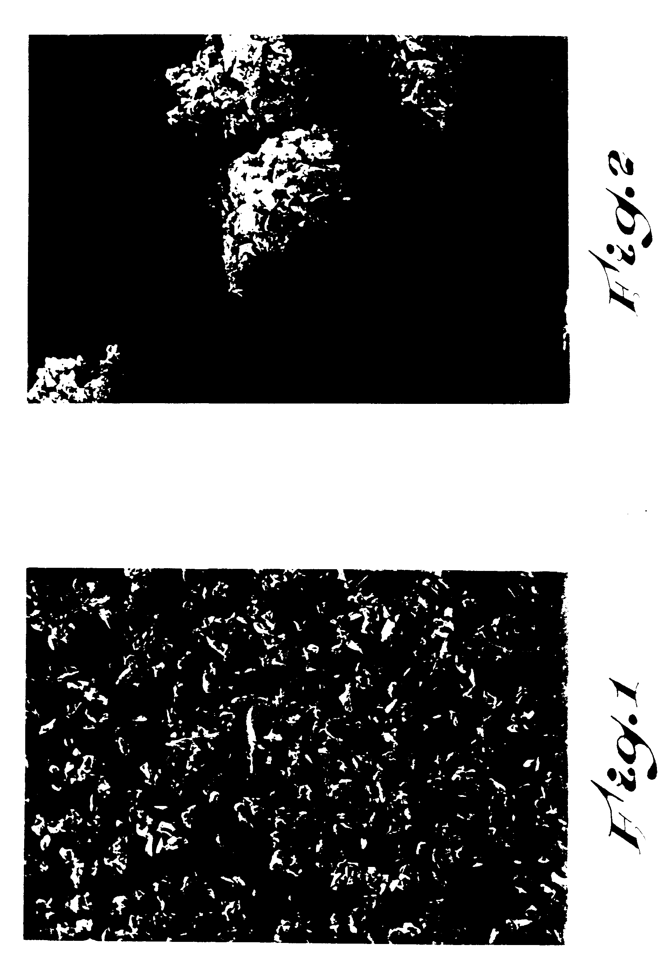

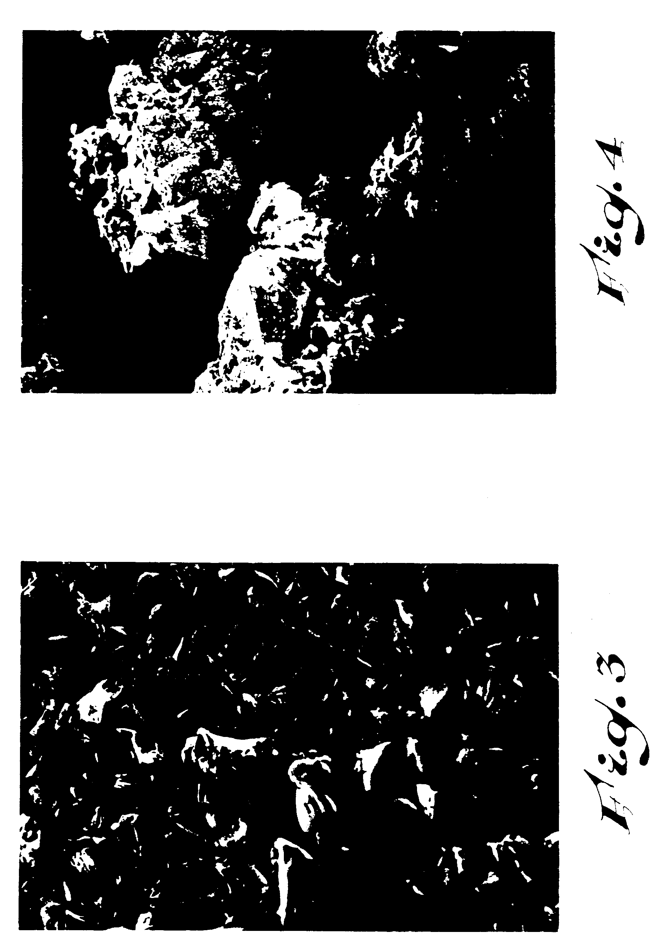

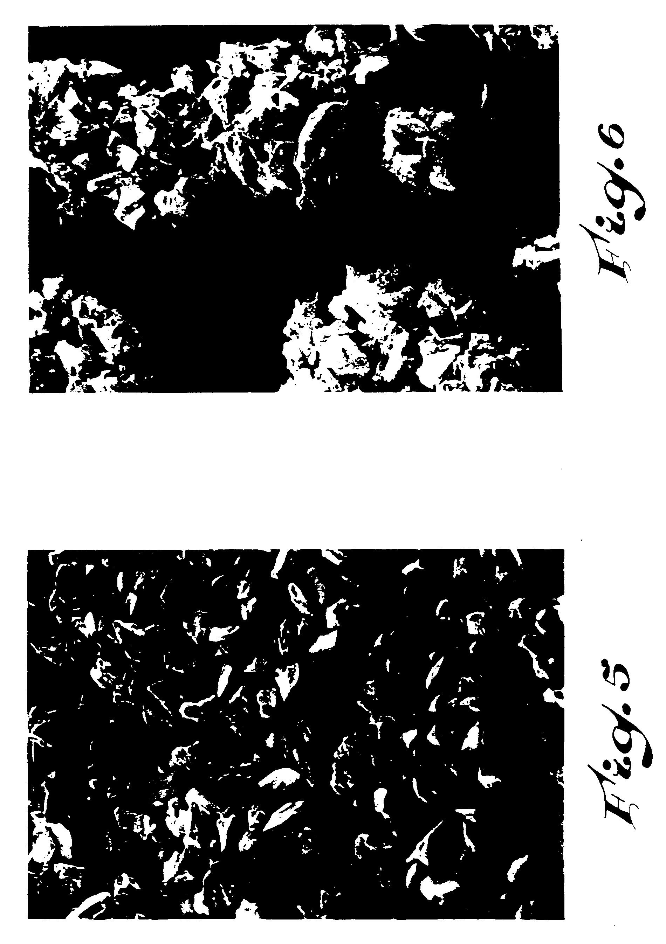

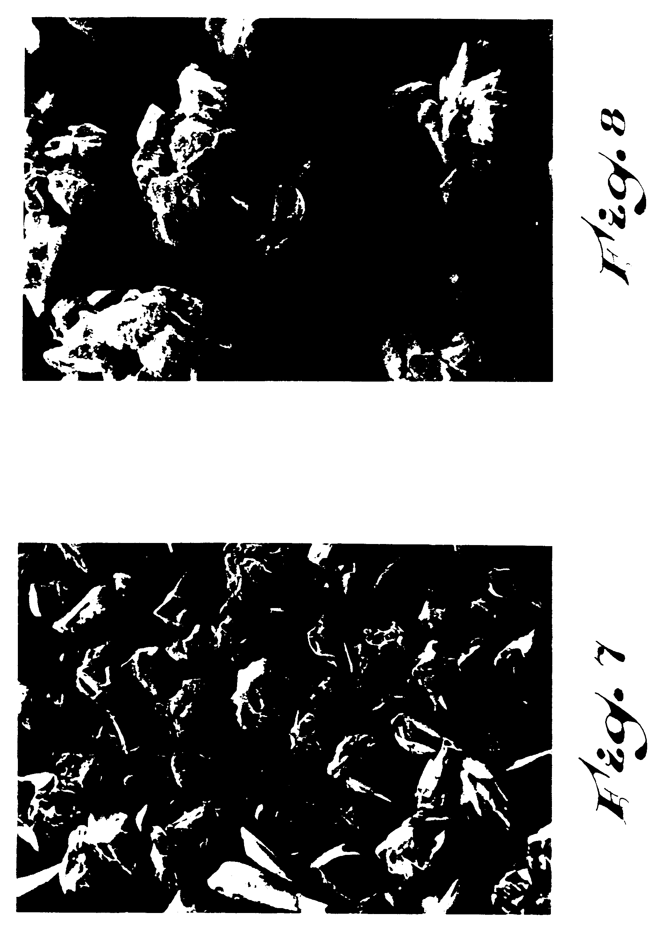

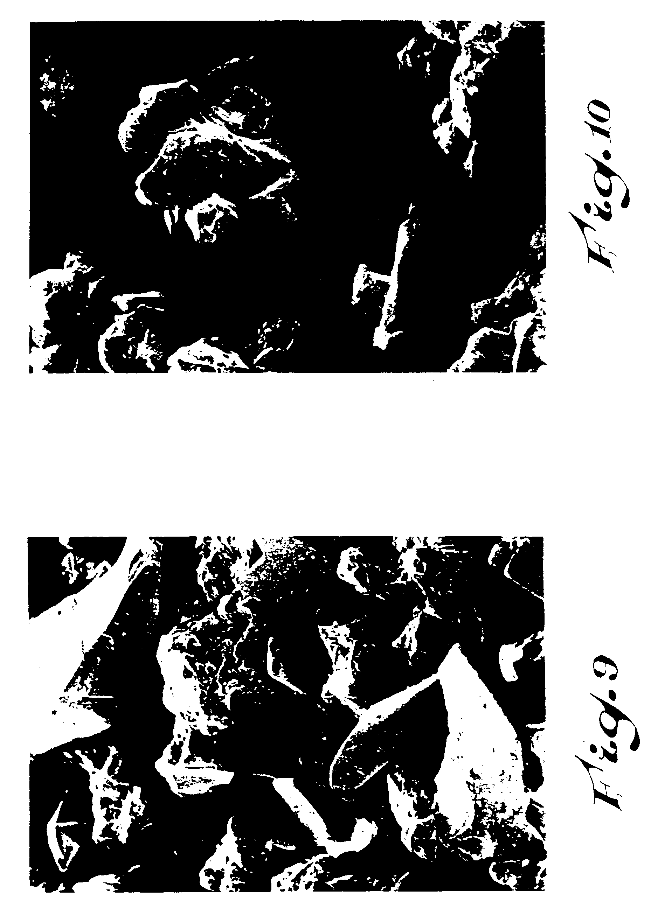

The Figures included herewith illustrate the invention by means of pairs of photomicrographs of the unassociated particles and the aggregates or clusters, of said particles. The actual invention is represented by the aggregates shown in Figures 2, 4, 6, 8 and 10. Figures 1, 3, 5, 7 and 9 depict the unassociated particles, and are not photographs of the present invention. Figure 1 is a photograph of unassociated water-swellable polymer particles having a size of 44 micrometers (325 mesh) and smaller and Figure 2 is a photograph of the aggregates or clusters of the unassociated particles having the particle size of 44 micrometers (325 mesh) and smaller. Figure 3 is a photograph of unassociated particles having a variety of sizes and Figure 4 is a photograph of the aggregates of those unassociated particles having varying sizes. Figure 5 is a photograph of unassociated polymer particles having a size of 88 to 44 micrometers (170 to 325 mesh) and Figure 6 is a photograph of the aggregates of the polymer particles having an unassociated particle size of 88 to 44 micrometers (170 to 325 mesh). Figure 7 is a photograph of unassociated polymer particles having a size of 149 to 88 micrometers (100 to 170 mesh) and Figure 8 is a photograph of the aggregates of the polymer particles having an unassociated particle size of 149 to 88 micrometers (100 to 170 mesh). Figure 9 is a photograph of unassociated polymer particles having a size of 297 to 149 micrometers (50 to 100 mesh) and Figure 10 is a photograph of the aggregates of the polymer particles having an unassociated particle size of 297 to 149 micrometers (50 to 100 mesh).

### A. The Water-Swellable Polymer Particles

The water-swellable or lightly crosslinked hydrophilic polymer particles useful in the present invention can be any of the known hydrophilic polymers which are capable of absorbing large quantities of fluids. Examples of such polymers include those disclosed in U S-A-4,833,222, 3,997,484; 3,926,891; 3,935,099; 4,090,013; and 4,190,562. Such hydrophilic polymers are prepared from water-soluble α,β-ethylenically unsaturated monomers such as mono and polycarboxylic acids and acrylamide and its derivatives.

The water-soluble monomers which are polymerized to form the water-swellable polymers of the present invention include those monomers listed in U S-A-4,833,222. Examples of such monomers include α,β-ethylenically unsaturated monomers such as mono and polycarboxylic acids.

### B. The Aggregates or Clusters

The water-swellable or lightly crosslinked hydrophilic polymer particles which benefit the greatest from being incorporated into the aggregates or clusters of the present invention are those unassociated particles which have a mesh size of less than 37 micrometers (400 mesh) and , preferably, from 88 to 37 micrometers (170 to 400 mesh). Such unassociated particles have been identified as causing gel blocking when such polymer particles are contacted with aqueous fluids. Gel blocking, it is thought, occurs when the polymer particles are small and fine, causing tight packing of a mass of unassociated particles. The particles of the mass of polymer are thought to be compacted to such an extent that swelling and gelling on the surface of the mass of polymer occurs when it is contacted with an aqueous fluid, blocking remaining aqueous fluid from freely flowing between the particles and being absorbed. These same unassociated particles do not exhibit gel blocking behavior when incorporated into the aggregates or clusters of the present invention.

The aggregates of water-swellable polymers are comprised of water-swellable polymer particles associated by being bound to other water-swellable polymer particles in a random packing configuration spacially distributed to allow aqueous absorption. The packing configuration of the polymer particles described herein as "random packing configuration spacially distributed to allow aqueous absorption" is illustrated in the photographs, Figures 2, 4, 6, 8 and 10. The Figures illustrate the particles of the aggregates or clusters as being bonded together in a random spacial distribution to allow for greater efficiency of aqueous absorption.

### C. The Aqueous Solution

The aqueous solution comprises an ethylenically unsaturated carboxylic acid monomer; the monomer is polymerizable with the water-swellable polymer of the present invention and includes all of those monomers described above as water-soluble monomers particularly acrylic acid, methacrylic acid, crotonic acid, and isocrotonic acid, alkali metal salts and ammonium salts thereof. Suitable polycarboxylic acids include maleic acid, fumaric acid, and itaconic acid. Suitable acrylamide derivatives include methacrylamide. The preferred monomers include acrylic acid and methacrylic acid and their respective salt forms such as alkali metal or ammonium salts.

Optionally a crosslinking monomer can be added to the aqueous solution. Organic compounds having two or more ethylenic groups copolymerizable with the water-soluble monomers can be used as the crosslinking monomers. Exemplary crosslinking monomers include diacrylate or dimethacrylate of ethylene glycol, diethylene glycol, triethylene glycol, propylene glycol, 1,4-butane diol, 1,5-pentane diol, 1,6-hexane diol, neopentyl glycol, trimethylol propane and pentaerythritol; triacrylates or trimethacrylates of trimethylol propane and pentaerythritol; tetracrylates or tetramethacrylates of pentaerythritol. N,N'-methylene-bis-acrylamide, N,N'-methylene-bis-methacrylamide and triallyl isocyanurate, and the like. The preferred crosslinking monomers for the present invention is trimethylolpropanetriacrylate.

Optionally, minor amounts of other water-soluble, unsaturated monomers may be present in the aqueous solution such as alkyl esters of the acid monomers. For example, methyl acrylate or methyl methacrylate may be present.

### D. The Polymer Particle Suspension

The inert hydrophobic liquid used to suspend the water-swellable polymer particles and the aqueous solution of monomer is usually an organic compound which is normally liquid at the conditions at which the polymerization process occurs. Operable liquids include hydrocarbons or substituted hydrocarbons. Preferred organic liquids are the halogenated hydrocarbons such as perchloroethylene, methylene chloride and the like, as well as liquid hydrocarbon having from 4 to 15 carbons per molecule including aromatic and aliphatic hydrocarbons and mixtures thereof, e.g., benzene, xylene, toluene, mineral oils, liquid paraffins such as kerosene, naphtha and the like. Of the foregoing organic liquids, the hydrocarbons are the more preferred, with aliphatic hydrocarbons being most preferred.

The particulate material comprising a hydrophobic character is an amorphous, highly oil-dispersible, approximately micrometer and sub-micrometer size, substantially water-insoluble particulate material. Typically, the size of the particulate material ranges from less than one to several micrometers in diameter. The particulate material is most preferably hydrophobic silicon dioxide, for example, the particulate material provided by the reaction of silica with polydimethyldichlorosilane. Other useful particulate materials include hydrophobic clays such as the cationic surfactant treated bentonite clays. An example of a hydrophobic clay is sold commercially as Bentone® 34 by N. L. Industries.

### The Process

Preparing the aggregates or clusters of the present invention requires suspending the aqueous absorbing polymer particles in the inert hydrophobic liquid. Typically, the weight ratio of polymer to liquid is not critical, however, for practical purposes the preferred ratio is in the range of from 1:10 to 10:1.

The aqueous solution includes an ethylenically unsaturated carboxylic acid monomer. The ethylenically unsaturated monomer solution is typically prepared by first dispersing the monomer in water. The monomer can be preneutralized and exist as a salt or as a mixture of the acid and the salt, however if the monomer is in acidic form, the pH of the solution should then be adjusted to between 4 and 7. The weight ratio of monomer is typically 1:10 to 5:10 monomer to polymer particles. Preferably the weight ratio of monomer is typically 2:10 monomer to polymer particles and the ratio of monomer to water is typically 0:10, preferably 4:10. Optionally, the aqueous solution may also contain a crosslinker, chelating agent and initiator. Therefore, the total monomer, if present, is present in the range of 15 to 45 weight percent based on total weight of the solution. The crosslinker is typically added in an amount of 0 to 5 weight percent based on the total weight of the monomer.

The amorphous highly oil dispersible substantially water-insoluble particulate material is suspended in an inert hydrophobic liquid. The aqueous monomer solution is then added to the particulate material to form a suspension of aqueous droplets or aqueous monomer droplets. The aqueous suspension or aqueous monomer suspension is then added slowly to the suspended polymer particles while the polymer particle solution is agitated and exposed to polymerization conditions. The polymerization temperature can range from 10°C to 100°C, depending upon initiators chosen.

The size of the aggregates or clusters formed will depend on the size of the polymer particles with which the process begins. However, a major contributor to the size of the aggregates or clusters is the size of the droplets of aqueous solution or aqueous monomer solution which are suspended in the inert hydrophobic liquid and added to the suspended particles solution. The droplet size is controlled by the amount of amorphous highly oil dispersible substantially water-insoluble particulate material present in the monomer solution. For example an aggregate of approximately 1000 micrometers can be formed when the droplets are approximately 50 micrometers in diameter. This is achieved if the particulate material is present in a ratio of approximately 0.3 to 2 percent based on the weight of total polymer present.

The aggregates or clusters can be filtered from the inert liquid, dried in an oven and crushed to a desirable size.

The hydration rate of the aggregate polymer particles is tested by evenly spreading 1.0 g of polymer particles over the bottom of a medium sized plastic boat (Fischer catalog 500 ct. #682-160-502). 30 Grams of a 0.9 percent sodium chloride solution is poured over the particles and a timer is simultaneously set. The hydration rate is that time required for the polymer to take up the solution and become stiff.

### Examples

The following examples illustrate the present invention.

### Example 1

In a one liter reactor 80 grams of Drytech® polymer (sodium polyacrylate polymer manufactured by the Dow Chemical Company), having a mixed particle size distribution, is mixed with 300 grams of Isopar M hydrocarbon (deodorized kerosene from Exxon). The dispersion is suspended using agitation. The monomer phase is prepared with a solution of 12 grams of acrylic acid; 0.05 gram of trimethylolpropane triacrylate; 0.05 gram of a chelating agent; 15.7 grams of water; 12 grams of a 50 percent solution of sodium hydroxide; and 0.1 gram of t-butyl hydrogen peroxide, suspended as droplets in a solution of 100 grams of Isopar M hydrocarbon and 0.25 gram of hydrophobic fumed silica sold as Aerosil® R-972 from Degussa. The aggregates or clusters are formed by adding the monomer phase to the reactor under constant agitation at 600 rpm. 20°C and under the flow of sulphur dioxide gas between 0.1 to 10.0 ppm/min. The aggregates are then separated from the hydrocarbon by filtration and then dried in a hot air oven at 100°C overnight.

The hydration rate of the clusters or aggregates of the invention is tested by evenly spreading 1.0 g of clusters over the bottom of a medium sized plastic boat (Fischer catalog 500ct. #682-160-502). Thirty grams of a 0.9 percent sodium chloride solution is poured over he particles and a timer is simultaneously set. The hydration rate is that time required for the aggregates to become stiff. The aggregates exhibited a hydration rate of 15 to 20 seconds with no visible gel blocking. A Comparative Example 1 of the Drytech® (The Dow Chemical Company) non-clustered polymer fines having mixed particle size distribution exhibited a hydration rate of greater than 10 minutes with visible gel blocking.

The absorbent properties are determined by the following procedures:
1. Filtered Free Swell Capacity (FFSC) is determined by allowing 1.0 g of the polymer aggregates or clusters to absorb its limit of 200 g of 0.9 percent sodium chloride solution in 30 minutes then filtered using a Buchner funnel and filter flask evacuated by an aspirator. The excess salt solution which is not absorbed and filtered is weighed and subtracted from the original 200 g to yield the filtered free swell capacity value.
2. Water Soluble Polymer Content is determined by extracting 1 g of absorbent polymer for 16 hours with 500 g of 0.9 percent sodium chloride. The swollen polymer is filtered off and the filtrate titrated with hydrochloric acid to determine the level of soluble polymer present.

The cluster or aggregates polymer composition of this example of the invention exhibits a FFSC of 26 g/g and a water soluble polymer content of 7 percent.

### Example 2

Polymer particles having a particle size of smaller than 44 micrometers (325 mesh) are associated similarly by the method used to produce the clusters of Example 1. The hydration rate, FFSC and water soluble levels of the aggregates are evaluated in a similar manner to those aggregates of Example 1. The hydration rate for the clusters of Example 2 is 15 to 20 seconds with no visible gel blocking, the FFSC is 28 and the water-solubles level is 7. Comparative Example 2, which consist of samples of the unassociated polymer particles having a particle size smaller than 44 micrometers (325 mesh), are evaluated in a similar manner to those of the Comparative Example 1. The samples of Comparative Example 2 exhibit gel blocking.

Example 2 and Comparative Example 2 illustrate that for particles of smaller than 44 micrometers (325 mesh), the aggregates or clusters of the invention exhibit a hydration rate of between 2 and 5 seconds whereas the unassociated particles of that same mesh size undesirably gel block.

## Claims

1. A process for preparing water-swellable polymer clusters comprised of water-swellable polymer particles being associated to each other in a random packing configuration and spatially distributed, which process comprises:
(a) suspending water-swellable polymer particles in an inert hydrophobic liquid to form a first suspension;
(b) suspending an amorphous highly oil-dispersible substantially water-insoluble particulate material in an inert hydrophobic liquid and adding an aqueous solution comprising an ethylenically unsaturated carboxylic acid monomer that is polymerizable with the water-swellable polymer to form a second suspension; and
(c) slowly adding said second suspension to said first suspension, while said first suspension is agitated and is exposed to polymerization conditions such that said particles are bonded to each other, whereby clusters of said water-swellable particles are formed.

2. The process of claim 1, wherein the ethylenically unsaturated monomer is acrylic acid, methacrylic acid, crotonic acid, isocrotonic acid, or an alkali metal or ammonium salt thereof.

3. The process of claims 1 or 2, wherein said aqueous solution additionally comprises a crosslinking monomer selected from: diacrylate or dimethacrylate of ethylene glycol, diethylene glycol, triethylene glycol, propylene glycol, 1,4-butane diol, 1,5-pentane diol, 1,6-hexane diol, neopentyl glycol, trimethylol propane or pentaerythritol, triacrylates or trimethacrylates of trimethylol propane or pentaerythritol, tetraacrylates or tetramethacrylates of pentaerythritol, N,N'-methylene-bis-acrylamide, N,N'-methylene-bis-methacrylamide or triallyl isocyanate.

4. The process of any of claims 1 to 3, wherein said inert hydrophobic liquid is perchloroethylene, methylene chloride, liquid aromatic or aliphatic hydrocarbons of 4 to 15 carbons per molecule or mixtures thereof.

5. The process of any of claims 1 to 4, wherein said particulate material aid is hydrophobic silicon dioxide.

6. The process of any of claims 1 to 5, wherein said ethylenically unsaturated monomer is present in an amount of about 15 to about 45 weight percent based on total weight of the aqueous solution.

7. The process of any of claims 1 to 6, comprising the additional steps of (d) drying the clusters, and (e) crushing the clusters.

8. An absorbent article, comprising:
(a) 50 to 98 percent by weight of said article of a hydrophilic fiber material; and
(b) 50 percent to 2 percent by weight of said article of aggregates of water-swellable polymers, prepared by (i) suspending water-swellable polymer particles in an inert hydrophobic liquid to form a first suspension; (ii) suspending an amorphous highly oil-dispersible substantially water-insoluble particulate material in an inert hydrophobic liquid and adding an aqueous solution comprising an ethylenically unsaturated carboxylic acid monomer that is polymerizable with the water-swellable polymer to form a second suspension; (iii) slowly adding said second suspension to said first suspension, while said first suspension is agitated and is exposed to polymerization conditions such that said particles are bonded to each other; and (iv) drying said aggregate.

## Patentansprüche

1. Verfahren zum Herstellen von wasserquellbaren Polymeragglomeraten aus wasserquellbaren Polymerteilchen, die in statistischer, räumlicher Packungsverteilung aneinander haften, durch
(a) Suspendieren wasserquellbarer Polymerteilchen in einer inerten hydrophoben Flüssigkeit, um eine erste Suspension auszubilden,
(b) Suspendieren eines amorphen, gut in Öl dispergierbaren, im wesentlichen wasserunlöslichen, teilchenförmigen Materials in einer inerten hydrophoben Flüssigkeit und Zugeben einer wäßrigen, ein ethylenisch ungesättigtes Carbonsäuremonomer, das mit dem wasserquellbaren Polymer polymerisierbar ist, enthaltenden Lösung, um eine zweite Suspension auszubilden, und
(c) langsames Zugeben der zweiten Suspension zu der ersten Suspension, während die erste Suspension gerührt wird und Polymerisationsbedingungen ausgesetzt ist, so daß die Teilchen miteinander verbunden und Agglomerate der wasserquellbaren Teilchen ausgebildet werden.

2. Verfahren nach Anspruch 1,
**dadurch gekennzeichnet**,
daß das ethylenisch ungesättigte Monomer Acrylsäure, Methacrylsäure, Crotonsäure, Isocrotonsäure oder ein Alkalisalz oder Ammoniumsalz derselben ist.

3. Verfahren nach Ansprüchen 1 oder 2,
**dadurch gekennzeichnet**,
daß die wäßrige Lösung zusätzlich ein vernetzendes Monomer enthält, ausgewählt aus Diacrylat oder Dimethacrylat von Ethylenglycol, Diethylenglycol, Triethylenglycol, Propylenglycol, 1,4-Butandiol, 1,5-Pentandiol, 1,6-Hexandiol, Neopentylglycol, Trimethylolpropan oder Pentaerythrit, Triacrylaten oder Trimethacrylaten von Trimethylolpropan oder Pentaerythrit, Tetraacrylaten oder Tetramethacrylaten von Pentaerythrit, N,N'-Methylen-bis-acrylamid, N,N'-Methylen-bis-methacrylamid oder Triallylisocyanat.

4. Verfahren nach einem der Ansprüche 1 bis 3,
**dadurch gekennzeichnet**,
daß die inerte hydrophobe Flüssigkeit Perchlorethylen, Methylenchlorid, flüssige aromatische oder aliphatische Kohlenwasserstoffe mit 4 bis 15 Kohlenstoffatomen pro Molekül oder Mischungen derselben ist.

5. Verfahren nach einem der Ansprüche 1 bis 4,
**dadurch gekennzeichnet**,
daß das teilchenförmige Hilfsmaterial ein hydrophobes Siliziumdioxid ist.

6. Verfahren nach einem der Ansprüche 1 bis 5,
**dadurch gekennzeichnet**,
daß das ethylenisch ungesättigte Monomer in einer Menge von etwa 15 bis etwa 45 Gew.-%, bezogen auf Gesamtgewicht der wäßrigen Lösung, vorhanden ist.

7. Verfahren nach einem der Ansprüche 1 bis 6,
**gekennzeichnet durch**
die zusätzlichen Schritte (d) Trocknen der Agglomerate und (e) Brechen der Agglomerate.

8. Absorbierender Gegenstand, enthaltend
(a) 50 bis 98 Gew.-% des Gegenstandes eines hydrophilen Fasermaterials und
(b) 50 bis 2 Gew.-% des Gegenstandes von Aggregaten wasserquellbarer Polymerer, hergestellt durch (i) Suspendieren wasserquellbarer Polymerteilchen in einer inerten hydrophoben Flüssigkeit, um eine erste Suspension auszubilden, (ii) Suspendieren eines amorphen, gut in Öl dispergierbaren, im wesentlichen wasserunlöslichen, teilchenförmigen Materials in einer inerten hydrophoben Flüssigkeit und Zugeben einer wäßrigen, ein ethylenisch ungesättigtes Carbonsäuremonomer, das mit dem wasserquellbaren Polymer polymerisierbar ist, enthaltenden Lösung, um eine zweite Suspension auszubilden, (iii) langsames Zugeben der zweiten Suspension zu der ersten Suspension, während die erste Suspension gerührt wird und Polymerisationsbedingungen ausgesetzt ist, so daß die Teilchen miteinander verbunden werden, und (iv) Trocknen des Aggregates.

## Revendications

1. Procédé de préparation d'agglomérats de polymère apte à gonfler dans l'eau, constitués de particules de polymère apte à gonfler dans l'eau, associées les unes aux autres suivant une configuration ramassée quelconque, distribuée dans l'espace, procédé qui comprend les étapes consistant à :
(a) mettre des particules de polymère apte à gonfler dans l'eau en suspension dans un liquide hydrophobe inerte, pour former une première suspension,
(b) mettre une matière amorphe sous forme de particules, pratiquement insoluble dans l'eau, très dispersable dans l'huile, en suspension dans un liquide hydrophobe inerte et ajouter une solution aqueuse renfermant un monomère acide carboxylique éthylèniquement insaturé, qui est polymérisable avec le polymère apte à gonfler dans l'eau, pour former une seconde suspension, et
(c) ajouter lentement ladite seconde suspension à ladite première suspension, tout en agitant ladite première suspension et en l'exposant à des conditions de polymérisation, de manière à ce que lesdites particules soient liées les unes aux autres, ce qui forme des agglomérats desdites particules aptes à gonfler dans l'eau.

2. Procédé selon la revendication 1, dans lequel le monomère éthylèniquement insaturé est l'acide acrylique, l'acide méthacrylique, l'acide crotonique, l'acide isocrotonique ou un sel de métal alcalin ou d'ammonium d'un tel acide.

3. Procédé selon la revendication 1 ou 2, dans lequel ladite solution aqueuse renferme en outre un monomère réticulant, choisi parmi le diacrylate ou diméthacrylate d'éthylèneglycol, de diéthylène glycol, de triéthylèneglycol, de propylèneglycol, de 1,4-butanediol, de 1,5-pentanediol, de 1,6-hexanediol, de néopentylglycol, de triméthylolpropane ou de pentaérythritol, les triacrylates ou triméthacrylates de triméthylolpropane ou de pentaérythritol, les tétraacrylates ou tétraméthacrylates de pentaérythritol, le N,N'-méthylène-bis-acrylamide, le N,N'-méthylène-bis-méthacrylamide, et l'isocyanate de triallyle.

4. Procédé selon l'une quelconque des revendications 1 à 3, dans lequel ledit liquide hydrophobe inerte est le perchloroéthylène, le chlorure de méthylène, un hydrocarbure liquide, aromatique ou aliphatique, ayant 4 à 15 atomes de carbone par molécule, ou un mélange de tels composés.

5. Procédé selon l'une quelconque des revendications 1 à 4, dans lequel ladite matière auxiliaire sous forme de particules est du dioxyde de silicium hydrophobe.

6. Procédé selon l'une quelconque des revendications 1 à 5, dans lequel ledit monomère éthylèniquement insaturé est présent en une proportion d'environ 15 à environ 45% en poids, par rapport au poids total de la solution aqueuse.

7. Procédé selon l'une quelconque des revendications 1 à 6, comprenant les étapes supplémentaires consistant à (d) sécher les agglomérats et (e) broyer les agglomérats.

8. Article absorbant, comprenant :
(a) 50 à 98% en poids, par rapport au poids dudit article, d'une matière fibreuse hydrophile, et
(b) 50% à 2% en poids, par rapport au poids dudit article, d'agrégats de polymères aptes à gonfler dans l'eau, préparés par (i) mise de particules de polymère apte à gonfler dans l'eau en suspension dans un liquide hydrophobe inerte, de façon à former une première suspension, (ii) mise d'une matière amorphe sous forme de particules, pratiquement insoluble dans l'eau, très dispersable dans l'huile, en suspension dans un liquide hydrophobe inerte et addition d'une solution aqueuse renfermant un monomère acide carboxylique éthylèniquement insaturé, qui est polymérisable avec le polymère apte à gonfler dans l'eau, de façon à former une seconde suspension, (iii) addition lente de ladite seconde suspension à ladite première suspension, tout en agitant ladite première suspension et en l'exposant à des conditions de polymérisation, de manière à ce que lesdites particules soient liées les unes aux autres, et (iv) séchage desdits agrégats.
